Europäisches Patentamt
European Patent Office
Office européen des brevets

(19)

(11) Publication number: **0 456 093 A2**

(12) # EUROPEAN PATENT APPLICATION

(21) Application number: **91107019.1**

(22) Date of filing: **30.04.91**

(51) Int. Cl.$^5$: **C07C 279/26**, A61K 31/155, A61K 9/70, A61K 7/00, A01N 47/44

(30) Priority: **10.05.90 US 521375**

(43) Date of publication of application: **13.11.91 Bulletin 91/46**

(84) Designated Contracting States: **AT BE CH DE DK ES FR GB GR IT LI LU NL SE**

(71) Applicant: **H.B. FULLER LICENSING & FINANCING, INC.**
**1100 North Market Street, Suite 780**
**Wilmington, Delaware 19890(US)**

(72) Inventor: **Olstein, Alan D.**
**1841 Walsh Lane**
**Mendota Heights, Minnesota 55118(US)**

(74) Representative: **Eisenführ, Speiser & Strasse**
**Balanstrasse 55**
**W-8000 München 90(DE)**

(54) **Biguanide derivatives and biocidal compositions containing them.**

(57) An antimicrobial compound comprising:

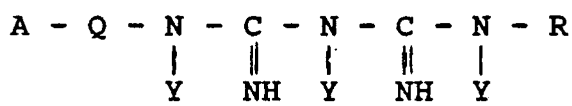

wherein Q = phenylene; Y = -H or -methyl;

R = $-(CF_2)_yCF_3$

wherein y = 1 - 20, $-(CH_2)_x-O-C_xH_{2X+1}$
wherein x = 1 - 25, $-(CH_2CH_2O)_n(CH(CH_3)CH_2O)_m(CH_2CH(CH_3)O)_p(CH_2CH_2O)_qT$
wherein T = -H or a branched or unbranched $C_{1-20}$ alkyl,
n, m, p, q = 0 - 99 and n + m + p + q $\geqq$ 1, -CHR'COOH wherein R' is a naturally occurring amino acid side chain, or $(CH_2)_xCOOH$; and A = -H, -OH, $NH_2$, $-CH_2 = CH_2$, $-(CH_2CH_2O)_n(CH(CH_3)CH_2O)_m(CH_2CH(CH_3)O)_p-(CH_2CH_2O)_qT$, an amine protein linkage, an amine saccharide linkage, a polyhydroxyamine linkage or -COOX wherein X = -H, an amino alcohol moiety, an amino alkoxy silane moiety, $-CONH(CH_2)_xCOOH$, or $-NHCH_2CH-(OX')_2$ wherein X' = methyl or ethyl. Such novel biocidal compounds may also be combined with proteins and polysaccharides and are stable in a variety of solutions and in a typical biocidal regimen or treatment locus. The biocidal compounds of the invention exhibit an improved antibacterial effect on both gram positive and gram negative bacteria, can have biocidal activity against a variety of other microorganisms including virus, yeast, molds, etc., and can have activity against multi-cellular pests.

EP 0 456 093 A2

## Fieldof the Invention

The invention relates to a novel biocidal, antibacterial or antimicrobial agent that can be used in typical biocidal, antibacterial or antimicrobial end uses. Such uses include surface sanitizing, food preservation, use in films such as adhesives, paints, or sealants, medical-surgical applications, general antiseptic uses, and others. The novel antibacterial agents of the invention can be used in the form of a solution in common solvents including water, alcohols, etc., and may be applied by dipping, wiping, spraying and the like as well as by being incorporated into the surface of application.

## Backgroundof the Invention

The growth of a variety of organisms on surfaces has been a problem for many years. Organisms taking the form of both unicellular and multi-cellular forms have caused infection in humans and other animals, spoilage of foods, caused chemical degradation of a variety of chemicals and have fouled surfaces in marine environments. In many areas the growth of unicellular and multi-cellular organisms has been a cause of substantial economic loss and physical hardship, even the loss of human life.

In aqueous and organic chemical systems, contamination with unicellular microorganisms can often degrade natural and synthetic polymers, adhesives, and lubricants causing substantial reduction in the desirable physical and chemical properties of any number of feedstocks.

On human and animal bodies, the infection of skin surfaces or wounds can cause significant illness, fever and potential loss of limbs or lives of affected individuals.

In application, studies have indicated that the contamination of both wet and dry surfaces with potentially pathogenic quantities of bacteria is widespread. Following a study of bacterial flora in 200 homes, Scott et al., J. Hyg. Camb., Vol. 89, 279 (1982), concluded that improved decontamination procedures are necessary, particularly at sites which are repeatedly wetted such as the surfaces of sinks, toilets, draining boards, stoves, washing machines and the like.

However, controlled in-use tests employing dilute aqueous detergents at kitchen and bathroom sites achieved no observable reduction of microbial contamination while application of aqueous hypochlorite and phenolic disinfectant compositions only produced a temporary reduction in microbial contamination. The evaluation of disinfectants in the domestic environment, Scott et al. J. Hyg. Camb., Vol. 92, 193 (1984), hypothesized that the rapid recontamination was due to fresh contamination of surfaces such as toilets and to the local multiplication of residual colonies at repeatedly wet sites, such as sinks.

In the marine environment, a variety of typically multi-cellular organisms form on hard surfaces such as ships, pilings, wharfs, quays, drilling rigs, sea water inlets, and screens among other areas. Such multi-cellular organisms must be removed mechanically if significant growth occurs that interferes with the operation of the associated surfaces. Such surfaces can be treated with chemical biocides, however, such materials to date tend to be toxic in nature and release materials such as organtin or organo-copper anti-fouling agents. Such agents are currently being discouraged in the domestic and world markets for environmental reasons.

The incorporation of various biocides into materials, such as adhesives, latexes, lubricants, either by physical entrapment, by ionic complexation or other method, has as yet not satisfactorily addressed the problem of providing potent prolonged antimicrobial action without significant release of toxic materials.

Therefore, a continuing need exists for an antimicrobial composition that can produce an ongoing biodegradable biocidal agent in sufficient concentration to provide a surface substantially free of organisms. A need also exists for an antimicrobial polymeric film capable of providing a surface with prolonged resistance to biological growth.

## BriefDescription of the Invention

The invention provides a composition that can protect surfaces or other compositions from microbial growth through the presence of biocidal materials. The present invention provides an antimicrobial composition comprising a monomer having the active functional group according to the following general formula:

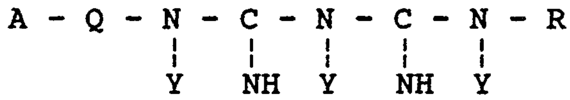

wherein Q = phenylene; Y = -H or -methyl; R = $-(CF_2)_yCF_3$
wherein y = 1 - 20, $-(CH_2)_xO-C_xH_{2x+1}$
wherein x = 1 - 25, $-(CH_2CH_2O)_n(CH(CH_3)CH_2O)_m$
$(CH_2CH(CH_3)O)_p(CH_2CH_2O)_qT$
wherein T = -H or any branched or unbranched $C_{1-20}$ alkyl,
n, m, p, q = 0 - 99 and n + m + p + q ≧ 1, -CHR'COOH wherein R' is a naturally occurring amino acid side chain, or
$(CH_2)_xCOOH$; and A = -H, -OH, $-NH_2$, $-CH_2 = CH_2$,
$-(CH_2CH_2O)_n(CH(CH_3)CH_2O)_m(CH_2CH(CH_3)O)_p(CH_2CH_2O)_qT$, an amine protein linkage, an amine saccharide linkage, a polyhydroxyamine linkage or -COOX wherein X = -H, $-NR_1R_2$ with $R_1$, $R_2$ = H or alkyl such as $-NH-CH_3$, an amino alcohol moiety, an amino alkoxy silane moiety, $CONH(CH_2)_x(COOH)$, or $-NHCH_2CH-(OX')_2$ wherein X' = methyl or ethyl. The compound of the present invention may also be grafted onto a biopolymer such as a protein or polysaccharide through a common amino acid bridge.

The biocidal compound can be dissolved in a suitable solvent and the resulting solution can be used directly as a liquid film-forming sanitizing composition. Alternatively, it may be used as an additive to materials, such as adhesives, lubricants, medical gauze, etc.

When used in an adhesive, it may reduce breakdown of the adhesive or substrate often caused by organisms. Oftentimes, adhesive layers from starch or other organic substances are attacked by organisms, resulting in a weakened adhesive. The organism breaks down the starch or other components, substantially compromising the adherent's strength. Furthermore, substrates, such as corrugated cardboard or other pulp-based material may also be attacked and broken down. The use of the present invention in the adhesive layer repels organisms from the adhesive layer and substrate, thus, resulting in a long-lasting bonded substrate. When used on a surface or dissolved in a solvent, the present invention displays biocidal properties. The liquid composition may be applied to a target surface by spraying, wiping, pouring, dipping and the like. The resultant surface is disinfected after solvent removal for a period of time and may be wiped, sprayed or contacted mechanically to further continue disinfection.

## DetailedDescription of the Invention

A surface that is coated with the antimicrobials of the invention will be sanitized for uses such as food preparation, medical or surgical use, or any other uses requiring a sterile environment. Coatings of the instant antimicrobial composition can be used on surfaces found in homes, hospitals, schools, in the work place, on marine surfaces, on the human body, etc. The biocidal compositions of the invention can also be used in compositions that are degradable by organisms. The biocidal agent may be used in adhesives, lubricants, latexes, coatings, or other compositions subject to attack from organisms.

The instant antimicrobial composition may be applied to a surface in a number of ways. The antimicrobial may be dissolved in a suitable solvent, preferably an organic or aqueous solvent. The antimicrobial solutions can be applied to a surface by a number of methods including wiping the composition onto a surface with a cloth or a pre-impregnated sponge, pouring the composition onto a surface and spreading it with a mop, squeegee sponge or cloth, dispensing the composition from a container equipped with a pump or spray mechanism, dispensing the composition as an aerosol using a propellant from a suitably pressurized container, dipping the surface into a solution of the material, and providing the composition in a sufficient concentration on a cloth or other absorbent carrier and packaging the premoistened carrier for disposable use.

Hard surfaces useful for coating with the instant biocidal agent include surfaces composed of refractory materials such as glazed and unglazed tile, brick, porcelain, ceramics, metals, glasses, and hard plastic such as formica, polystyrene, vinyls, acrylics, polyesters, polycarbonates, polyaramides and the like. The liquid compositions are preferred, preferably coated at a thickness sufficient to form a residual film of about 0.01-5 millimeters in thickness prior to solvent evaporation. As an antimicrobial agent in bulk materials, the material can be used at a concentration of about 0.001 wt-% to 1 wt-%.

## BiocidalAgent

The preparation of the biocidal agent of the invention can be made by preparing an asymmetric biguanidine compound comprising an ether portion on one end of the biguanidine and an end cap group forming the balance of the asymmetric guanide structure. The synthesis of the asymmetrical biguanide material begins with the synthesis of a monoguanide compound of the formula (I):

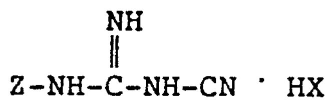

(I)

wherein X is a halide and Z may be any variety of alkyl or aryl moieties including phenyl having any variety of reactive hydrogen containing groups positioned para in relation to the guanide chain. This compound is generally formed by reacting an amine which is either mono- or di-substituted with a dicyanamide salt such as sodium dicyanamide, $NaN(CN)_2$.

A biguanide compound is synthesized having the formula (II):

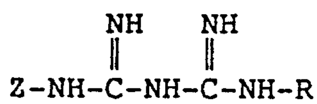

(II)

wherein Z is any variety of alkyl or aryl moieties and R is any variety of alkyls, ether alkyls, ether amines among other moieties. Preferably, Z is a phenyl having an additional reactive moiety such as a hydrogen, amine, hydroxyl, carboxyl, olefin or other polymerizable group positioned para with relation to the biguanide structure. Preferably, R is an alkyl, alkyl ether, ether amine, carboxylic acid or alkyl carboxylic acid moieties.

The biguanide compound is generally synthesized by reacting the monoguanide compound with a second mono- or di-substituted amine. The resulting product is a biguanide substituted compound. The end group Z is preferably phenyl and may have any number of polymerizable or nonpolymerizable groups attached to either the ortho, meta, or para position, with the para position preferred.

Generally the compound of the present invention has an ether amine in the R position as depicted in formula (III):

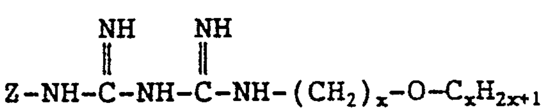

(III)

wherein x = 1 - 25. Alternatively R may be a moiety having any number of moles of ethoxylation, propoxylation or mixtures thereof in block or heteric form.

Amines that can be reacted with the dicyanamide compound to form the biguanide-substituted compounds include mono and diamines having functional groups that can be reacted to form the ether compounds of the present invention. Monoamine and diamine compounds useful in forming the compound of the present invention include mono or dialkyl amines having a $C_{1-12}$ alkyl group, such as methyl amine, ethylamine diethylamine, dihexylamine, etc., a carboxylic acid containing substituents such as aminobenzoic acid, alpha amino acids, 6-aminohexanoic acid, 11 amino undecanoic acid and the like.

The preferred amine for use in the present invention is an amine of formulae (IVa) and (IVb):

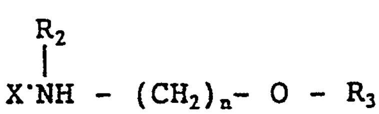

(IVa)

or

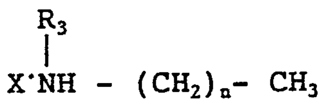

(IVb)

wherein n = 1 - 12, x is a halogen, $R_2$ is H or a $C_{1-24}$ branched or unbranched alkyl group and $R_3$ is H or a $C_{1-5}$ branched or unbranched alkyl. The reaction product formed from these amines and the monoguanido compound have corresponding formulae (Va) and (Vb):

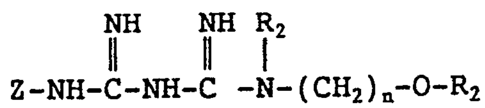

(Va)

or

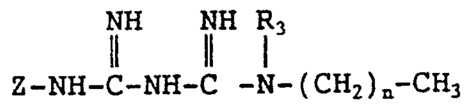

(Vb)

wherein the groups are as defined above.

The active ether amine portion of the asymmetrical biguanide structure can be formed by reacting the substituted biguanide compound at the nitrile functionality with an ether amine compound or a perfluoro alkyl amine compound shown above. The preferred ether amine compounds comprise a compound of the formula (VI):

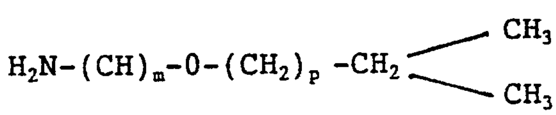

(VI)

wherein m is an integer of 1 to 6 and p is an integer of 3 to 21. The most preferred ether amine compounds are compounds of the formula (VII):

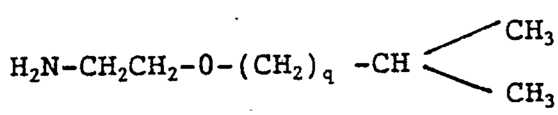

(VII)

wherein q is an integer of 4 to 16.

In the preferred perfluoro alkylamine compound, the compounds have the formula shown in (VIII):

$$-(CF_2)_y-CF_3 \qquad \text{(VIII)}$$

wherein y = 1 - 20.

The R moiety may also be an amino acid moiety. Preferred amino acid moieties include groups having the structure -CHR'COOH wherein R' is any naturally occurring amino acid side chain. Preferred amino acids include glycine, alanine, serine, threonine, asparagine, glutamine, and histidine wherein R'is the respective side chains of these amino acids.

Turning to the other end of the compound, Z may be viewed generally as phenyl having additional functional moieties positioned on the ring, preferably para to the biguanide structure as can be seen in the formula (IX):

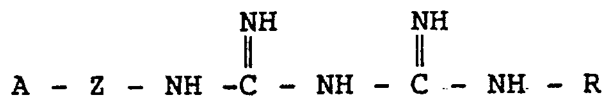

(IX)

wherein A may be -H, -OH, $-NH_2$, $-CH_2=CH_2$, carboxyl, any amino alcohol, amide, any amino alkoxy silane, ether amines, or complex amide structures having an amine peptide, an amine protein linkage, or a simple or complex sugar moiety thereon. preferred amino sugars are 2-dioxy-2-amino-D-glucopyranose and polyhydroxy amine. Generally, amino alkoxy silanes also useful in the present invention include moieties having the formula (X):

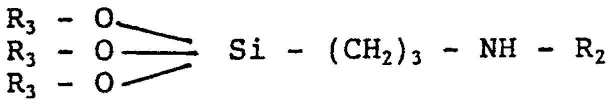

(X)

wherein the groups are as defined above. Preferred amino alkoxy silanes include γ-amino propyl trialkoxy silanes such as γ-amino propyl trimethoxy silane or γ-amino propyl triethoxy silane, or γ-amino propyl t-butyloxy, dimethoxy silane.

## Applications

Generally, the compound of the present invention may be used in any number of different forms depending upon the functional moieties contained in the various positions around the molecule. Generally the compound of the present invention is present in a concentration adequate to provide effective antimicrobial action across the intended spectrum. These concentration ranges may vary from about 0.001 wt-% to 5 wt-%, preferably from about 0.001 wt-% to 1 wt-% and most preferably from about 0.001 wt-% to 0.1 wt-%. The compound of the present invention may be used within film formers such as adhesives, in surface cleaners, in topical scrubs, in coating compositions, personal care products, pump or aerosol sprays, sealants and the like, among other compositions.

In film forming compositions such as adhesives, the compound of the present invention may be formulated directly into either a solvent based or aqueous system by simple mixing. The compound of the present invention may be used to thwart the growth of deleterious microorganisms in adhesives such as for example, hydrogels comprising polysaccarides which are largely susceptible to microbial contamination.

Other adhesive compositions such as pressure sensitive adhesives may also comprise the compound of the present invention as an antimicrobial agent. In this instance, acrylates such as ethyl or hexylmethacrylate are formulated into a latex emulsion. The compound is then added neat to the composition. Generally, the compound of the present invention may be used at concentrations which provide adequate antimicrobial activity across the intended spectrum. Generally, this will be in concentrations ranging from 0.1 to 5 % by weight while greater or lesser concentrations are also possible. The film forming agents comprising the compound of the present invention may be used in systems such as elastic bandages, bandage adhesives, as well as any number of other adhesive compositions or film agents.

The compound of the present invention may also be used in antimicrobial films such as teat dips. In this instance, the compound of the present invention would be formulated into an aqueous composition having a high viscosity comprising other agents such as surfactants, carboxylic acids, and the like. Here again, the compound of the present invention may be used in concentrations intended to provide broad spectrum antimicrobial activity.

The compound of the present invention may also be used in aqueous or organic solutions which are intended to be applied as surface cleaners to porous and nonporous substrates. In this instance, the surface cleaner may comprise any number of constituents including buffers, surface active agents, defoaming agents, and the compound of the present invention at a concentration which is effective in providing a broad spectrum of antimicrobial action or antiviral action.

The compound of the present invention may also be used in topical scrubs which comprise surfactants, simple fatty acids, conditioners, foam builders such as alkanolamides, buffers, etc. In this instance, the compound of the present invention may have a dual function of preserving the scrub or cleanser and acting as a topical antimicrobial to clean the skin of unwanted contaminants. The concentration of the compound of the present invention may be adjusted to provide, here again, any range of antimicrobial action appropriate for the intended use, generally using the concentration of a compound as provided above.

The compound of the present invention may also be used in coating systems of any non or nonwoven fibers, porous or nonporous substrates, including paper, ceramics, wood, etc., as coatings for filters for ultrafiltration in applications such as pharmaceutical purification, blood transfusions, hemodialysis, as well as plasma phoresis and the like. In this instance, the coating may be applied through dip or spray applications. The coating composition may generally comprise any number of different constituents including silanes, organic solvents such as ethanol, methanol, propanol, and the active monomer of the present invention.

Generally, the coating composition of the present invention may be also used on substrates which are silicon reactive such as polyurethanes, polytetrafluoroethylene, polyvinyl chloride, or polyvinylidene fluoride, and the like to provide a system which by its silicon reactivity combines with the silane constituent in the coating composition to effectively lock the active ingredient into the coating compound across the surface of the substrate. Here again, the concentration of the compound would vary depending upon the intended application and the spectrum of activity desired of the coating agent.

The compound of the present invention may also be used in personal care products such as hair and skin conditioners or creams. Such compositions may be desirable for treating various dermal conditions resulting from antimicrobial or viral agents. In this instance, these compositions may comprise any number of constituents including water, long chain fatty acids, conditioning agents, sequestrants and the like. The compound of the present invention would generally be used in concentrations ranging from 0.1 wt-% to 5 wt-% depending upon the condition which is to be addressed.

The compound of the present invention may also be used in pump or aerosol spray compositions to be applied as a sanitizing or disinfecting coating or solution. In this instance, the compound of the present invention is a highly appropriate constituent as it generally has a molecular weight highly suitable for pump or aerosol spray application. In addition to the compound of the present invention these compositions would generally also comprise water, sequestrants if needed, and depending upon the system of application, any variety of commercially available propellants.

The compound of the present invention may also be used in any variety of sealants.

One application among many of the compound of the present invention is its use in biomedical sealants appropriate for application to medical conditions such as colostomies. For example, the compound of the present invention may be used in adhesives which are intended to seal a colostomy bag to a patient's port or stoma. In this instance the need for an antimicrobial to maintain the consistency of the adhesive is especially pronounced given the passage of various fluids and materials from the intestines of the patient which may have any variety of antimicrobials therein.

Also, the compound of the present invention may be used in sealants such as caulks, grouts and mortars found in any variety of applications which may be subjected to antimicrobial attack. For example, sealants used in cement docks at ocean fronts or lake fronts are often subjected to degradation and fouling by micro and macroorganisms. Sealants comprising the compound of the present invention would not only deter the formation and incrustation by macroorganisms but also maintain the consistency of the sealant. Exemplary compositions are those again comprising any number of acrylics including methacrylate, methylmethacrylate, hydroxyalkylacrylate, hydroxyalkylmethacrylate, butylacrylate, hexylacrylate, cyclohexylacrylate, cyclohexymethacrylate, (2-hydroxyethyl)acrylate, (2-hydroxyethyl)methacrylate, (3-hydroxypropyl)methacrylate, (3-hydroxylpropyl)acrylate,
(dimethylamino-ethyl)methacrylate,
(pipiridinoethyl)methacrylate,
(morpholinoethyl)methacrylate and the like. These acrylics are generally preformed into a latex emulsion system with the active compound of the present invention blended in to the emulsion after it is formed.

As can be seen, the compound of the present invention may be used to obtain enhanced antimicrobial activity in any variety of compositions which are used in various applications including health care, food preparation, sanitation and cleaning, the construction industries, and the personal care products industry. However, these compositions and applications should not be looked at as limiting, rather as exemplary of the versatility of the compound of the present invention.

The following Examples illustrate the preparation of the compound of the present invention including its use in certain final applications including personal care products, medical products, and antimicrobials generally. These Working Examples should be viewed simply and purely as illustrative and not as limiting of the present invention.

## WORKINGEXAMPLES

Example I

Synthesis of 4-Cyanoguanidino Phenol

The synthesis was initiated by charging 100 milliliters of water containing 5.3 grams of sodium dicyanamide ($NaN(CN)_2$ 0.06 mole) into a 200 ml flask having a reflux condenser and nitrogen purge. After the dicyanamide compound was dissolved, 8.7 grams of paraminophenol chloride (0.6 mole) was added to the mix. The mixture was refluxed at ambient pressure for 30 minutes. At the end of the reaction a purple fluffy mass was observed in the reflux container which was washed with 2 liters of water, 1 liter of acetone and then dried under vacuum. The IR spectrum in (Nujol-Mull) of the sample is consistent with the structure:

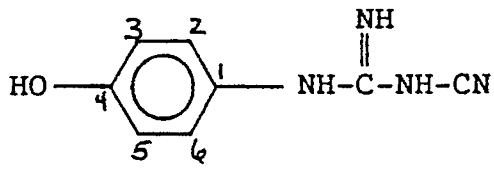

$C_{13}$NMR showed two sharp peaks located at about 3430 $cm^{-1}$ and 3370 $cm^{-1}$ represent NH and OH groups. Also C = N and C≡N are observed. Both proton and carbon 13 nuclear magnetic resonance spectra obtained on the compound are entirely in agreement with the structure shown above. The table below gives carbon number assignments and C-13 chemical shifts with respect to deuterated DMSO at 39.79 ppm.

**Table 1**

C-13 Chemical Shift Table

| Carbon Number | $\delta^{13}C$, ppm |
|---|---|
| 1 | 160.5 |
| 2 | 155.1 |
| 3 | 129.2 |
| 4 | 125.2 |
| 5 | 118.2 |
| 6 | 115.8 |

Example II

Synthesis of N-1 Hydroxyphenol, N-5

Oxypropyl Isononyl Biguanidine

This compound was synthesized by adding 25 milliliters of dimethylformamide and 5 grams of paracyanoguanidino phenol and 6.68 grams of oxypropyl-isononylamine hydrochloride into a 50 ml round bottom flask equipped with a reflux condenser and nitrogen purge. The mixture was maintained at 155° C. for 3.5 hours while the reaction was monitored using thin layer chromatography. One major compound appeared during the reaction. The contents of the reaction flask were diluted with 300 ml of 0.3 molar sodium carbonate and extracted with toluene to remove the unreacted amine. The product formed an insoluble purple-black mass at the water toluene interface. This material was collected and dissolved in 2% acetic acid. The product was then extracted into butanol and evaporated under vacuum. The residue is a purple oil miscible in water, DMS, ethanol and methanol.

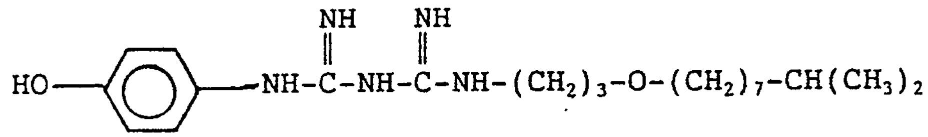

Infrared analysis showed $CH_2$ stretch at 2950 $cm^{-1}$ and 288 $cm^{-1}$; imine absorption at 1675 $cm^{-1}$; phenyl absorption at 1510 $cm^{-1}$; C-$CH_3$ absorption at 1455 $cm^{-1}$ and 1380 $cm^{-1}$; and aliphatic ether absorption at 1110 $cm^{-1}$ and 1170 $cm^{-1}$.

An NMR study of the compound was then undertaken which showed that the compound resulting from the synthesis was largely as expected. The nitrile carbon peak was absent as expected.

Example III

Preparation of Perfluoro Alkyl Derivative

Perfluorooctyl amine was prepared from perfluorooctanoic acid by first diluting 363 grams of the acid in 50 ml of methanol in the presence of 85 grams of $BF_3$ (30w/v(methanol)) and stirring at reflux for 6 hours. The mixture was then cooled and transferred to a separatory funnel whereupon 50 ml of $H_2O$ was added and the lower product phase (excluding a solid) precipitate was separated and dried over a $Na_2SO_4/CaCl_2$ bed. The crude ester was then added to a 3-neck round bottom 1 liter flask along with 350 milliliters of Freon 113. The solution was slowly stirred and anhydrous ammonia was added at a rate slow enough to prevent ammonia reflux. The reaction ended when the ammonia began to reflux and the flask began to cool. the white solid precipitate was collected by filtration and washed with cool Freon 113 and then dried. The mother liquor was evaporated to dryness and dessicated to provide additional perfluorooctyl amide. The amide (142.2 grams) was then combined in an oil bath with 205 grams of $P_2O_5$ and56.7 grams super cell under stirring at 180° C. in a 1-liter flask. The product was distilled over 1 hour and after about 6 hours the product was collected by vacuum filtration. Then the nitrile product (250 grams $C_7F_{15}CN$) was added drop wise over a 30 minute period to a well stirred refluxing slurry of $NaBH_4$ and diglyme. The resulting mixture was stirred an additional two hours at reflux, cooled, and poured slowly onto 700 milliliters of ice/water. Foaming occurred. The mixture was cooled in an ice bath and about 700 milliliters of 20% aqueous sodium hydroxide was added until the pH reached 11. The mixture was extracted with isopropylether. The extract was then dried over anhydrous calcium sulfate and stripped giving the crude $C_7F_{15}CH_2NH_2$.

Then 4.8g (0.020 mole) of p-cyanoguanidine benzoic acid was dispersed in 25 mil of tetrahydro-furan and 9.8g (0.020 mole) of perfluoroctylamine hydrochloride was added. The reaction mixture was brought to reflux and after 20 minutes the solid dissolved. A white crystalline product gradually crystallized out of solution during the course of the reaction, 60 minutes. Consumption of the nitrile was monitored by IR at 2200 $cm^{-1}$ until it was no longer evident. The crystalline product was filtered and washed with isopropanol.

Infrared analysis showed loss of nitrile at 2200 $cm^{-1}$, aromatic carbonyl acid at 1725 $cm^{-1}$, a strong imine absorption at 1620 $cm^{-1}$, carboxylic acid salt at 1580 $cm^{-1}$ and aliphatic fluoro alkane at 1120, 1150, 1170 $cm^{-1}$. These data strongly support the structure assignment to the following structure:

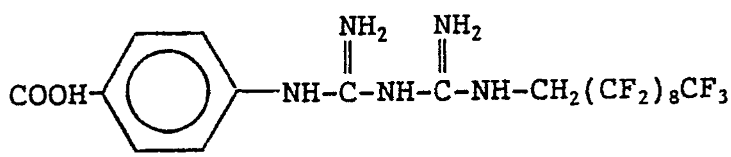

Example IV

Synthesis of a monoether benzoate biguanidine was initiated by first preparing isododecyl oxypropyl cyanoguanidine. First, 21g, 0.144 mole of S,S-dimethyl cyanoimide dithio carbonate was dissolved in 50 ml isopropanol and 15 ml aqueous ammonia. The reaction mixture was stirred overnight at room temperature. A white crystalline product was collected and recrystallized from isopropanol. The composiiton had a melting point of 177° C (175 - 176° C) and infrared analysis disclosed nitrile at 2200 $cm^{-1}$, thioether at 1430 $cm^{-1}$. The N-cyano, S-methyl isothiourea prepared above was then warmed in 15 ml ethanol to dissolve

6.0g (0.0513mole) One equivalent of the ether amine isododecyl oxypropylamine, 12.97g (0.051 mole) was added into the section mixture and stirred overnight at room temperature. The product, a pale yellow oil, was diluted into one volume of ethyl acetate and washed 3 times with 2 volumes water, and aqueous sodium bicarbonate. Infrared analysis disclosed evidence of nitrile at 2200 $cm^{-1}$, imine at 1640 $cm^{-1}$, amidine at 1580 $cm^{-1}$, and aliphatic ether at 1110 $cm^{-1}$.

The $C_{13}$NMR showed an imine carbon at 161.5 ppm, nitrile carbon at 118.5 ppm, ether carbons at 67.8 and 68.5 ppm. Aliphatic carbons were observable at 20 - 40 ppm. Additionally, there is no evidence of the presence of a thioether methyl carbon. These data support the conclusion that the desired alkyl ether cyanoguanidine was obtained.

Preparation of the monoether benzoate biguanide was initiated by dissolving 10g (0.031 moles) of the alkyl monoether cyanoguanidine in 20 ml THF and adding 5.1g (0.031 mole) of ethyl amino benzoate and 5 mil con. HCl. Water (10 ml) was then added to solubilize the reactants and the mixture was brought to reflux. After two hours, the nitrile peak at 2200 $cm^{-1}$ was essentially consumed. The product was worked up by extraction into ethyl acetate from dilute aqueous HCl (pH 2.0).

Infrared analysis disclosed ester carboxyl at 1720 cm $^{-1}$, 1280 $cm^{-1}$, strong imine absorption at 1640 $cm^{-1}$ and aliphatic ether at 1110 $cm^{-1}$.

The $C_{13}$ NMR analysis disclosed the imine carbons at 160.4 ppm and 165.6 ppm respectively; aromatic carbons at 113.3 ppm, 131.8, 123.8, 143.8 ppm ester carboxyl at 166.4 ppm, ether carbons at 59.6 ppm and aliphatic methylene carbons ranging from 15 ppm to 40 ppm. The aforementioned IR and NMR analysis are consistent with the following structure.

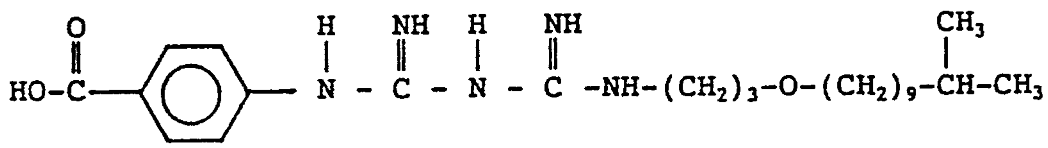

Example V

A dipeptide cyanoguanide was synthesized by dissolving 4.2g of p-aminobenzoyl-β alanine (0.02 mole) in 20ml methanol and 1.0 equiv. HCl (1.7ml) was added. The solvent was evaporated to yield the HCl salt. The compound was dissolved in 20ml water and adjusted pH to about 3.0 buffered with 0.1 equivalent sodium acetate (0.2g) and warmed to 60°C for additional fifteen minutes. A theoretical yield of 79% was obtained.

Infrared analysis showed evidence of nitrile at 2200$cm^{-1}$, amide at 1720$cm^{-1}$, and imine absorption at 1650 $cm^{-1}$ as well as carboxylic acid absorption 3000 - 2500$cm^{-1}$ (broad) and 1440$cm^{-1}$. These data evidence the desired dipeptide cyanoguanidine:

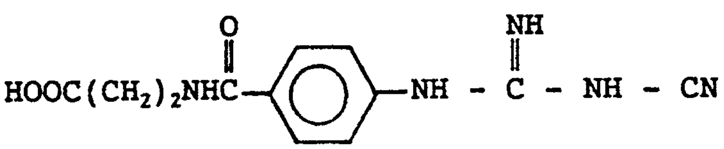

Example VI

An ether amine biguanide was synthesized by dissolving 5.0g p-cyanoguanidine benzoyl β-alanine in 25ml DMF, 3.95gm one equivalent of ether amine $(NH_2H_2)_3(O)_2(C_{13}$ alkyl)), (Exxon PA-17) was acidified and the pH was adjusted to pH 3.5. The preparation was predried overnight over a 4°A mol sieve. The solution was then brought to reflux for 3.5 hours. Product formation was monitored by infrared and thin layer chromatographic analysis. The product was worked up by extraction from aqueous solution with three volumes of hexane.

Infrared evidence of incorporation of ether amine showed aliphatic absorbances at 2900$cm^{-1}$, loss of nitrile at 2200$cm^{-1}$, and aliphatic ether absorption at 1100$cm^{-1}$. $C_{13}$NMR spectrum was consistent with the IR interpretation, i.e. acid and amide resonances were present 173.2 ppm, and 170.5 ppm respectively.

Ether carbons are observable at 67.9 ppm. These data are consistent with the following structure:

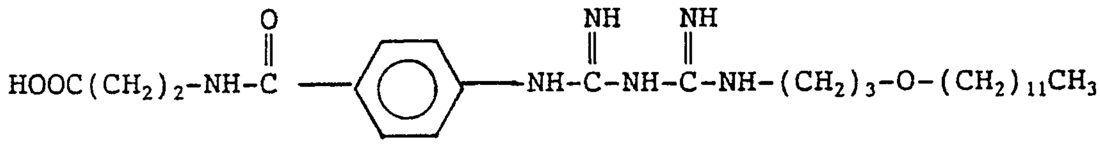

Example VII

An N-allyl amide of an acidic peptide biguanide was synthesized by dissolving 15g, (0.031 mole), of β-alanyl benzoyl biguanide in 35ml methanol, after which 6.3g, (0.030 moles), of dicyclohexylcarbodiimide and 1.76g (0.032 mole) of allylamine were added. The reaction mixture was stirred for 1 hour at room temperature. The insoluble dicylcohexyl urea was filtered off and the supernatant was extracted with 3 volumes of hexane.

$C_{13}$NMR showed evidence of loss of acid carbon at 173 ppm and the appearance of an alpha and beta unsaturated amide at about 166 ppm. This data coupled with the thin layer chromatograph and infrared spectrum, showing evidence of a substituted olefin at 990 ppm supports the conclusion that the desired alkyl amide was obtained:

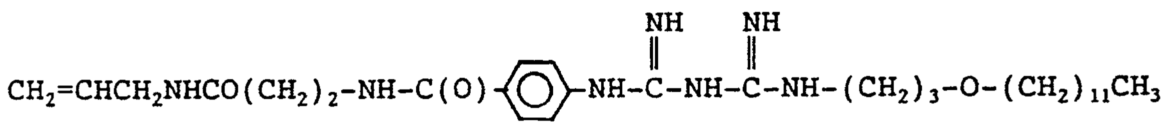

Example VIII

Synthesis of an aniline biguanidine was initiated by dissolving 5.0g of p nitro trifluoroacetyl acetanilide, (0.0203 mole) in 50 ml THF and 4.2g of palladium-polyethylenimine catalyst (Aldrich) was added with 29 ml 97% formic acid. The reduction mixture was agitated at 45° C. for 18 hours whereupon the catalyst was filtered off and the solvent was evaporated. A small amount of concentrated HCl was added to convert the aniline to its hydrochloride salt which was recrystallized from THF. Infrared analysis showed evidence of amine hydrohalide at 2550 $cm^{-1}$, TFA-amide doubled at 1710 $cm^{-1}$, 1750 $cm^{-1}$, strong aromatic amine 3340 $cm^{-1}$ and 850 $cm^{-1}$. Some residual aryl nitro group is detectable at 1510 $cm^{-1}$. TLC shows very little residual aromatic nitro, >90% aromatic amine. Then 4.8g, 0.019 mole of the p-amino trifluoro acetanilide hydrochloride prepared above was dissolved in 15 ml THF and 6.2g of the isododecyl monoether cyanoguanidine from Example IV was added to the reaction mixture. The solution was brought to reflux and maintained for 2.5 hours. The nitrile absorption was monitored at 2200 $cm^{-1}$, and concomitant with its disappearance a more prominent imine peak appeared at 1620 $cm^{-1}$. TLC confirms consuption of starting aromatic amine. The infrared analysis shows imine absorption at 1620 $cm^{-1}$ aromatic amine at 1520 $cm^{-1}$, 850 $cm^{-1}$, aliphatic ether at 1150 $cm^{-1}$. 13-C NMR spectroscopy showed evidence of imine carbons at 160.5 ppm, 166.5 ppm respectivley, aromatic carbons 2,6 and 3,5 at 113.2 and 126.2 ppm respectively, aniline C-1 at 156.5 ppm and the ether and methylene carbons are in their associated positions within the spectrum. These data support the conclusion that the aniline type biguanidine was obtained.

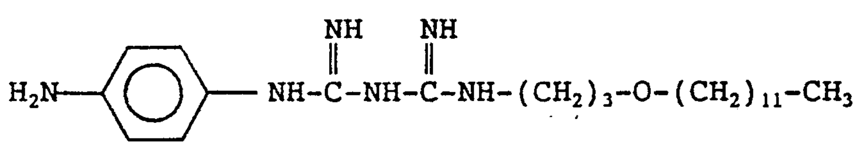

Example IX

Preparation of polyether alkanol phenolic biguanidine was initiated by dispersing 5.5g, 0.243 mole of p-cyanoguanidine phenol in 24.5g of polyether alkanolamine. Sufficient HCl was added to bring the pH to about 2.0. The mixture was heated in a three-necked mechanically stirred vessel under nitrogen to 85°C. The reaction progress was monitored by IR following the disappearance of the nitrile peak at 2200 $cm^{-1}$. The cyanoguanidine dissolved in the amine at about 50°C and after ten minutes the nitrile was undetectable in the IR.

IR showed evidence of phenol at 3100 $cm^{-1}$, aliphatic ether at 1100 $cm^{-1}$ and imine absorbance at 1670 $cm^{-1}$.

A $C_{13}$ NMR spectrum was then run on the resulting monomer. The NMR spectrum was then run with the results showing imine carbons observable at 158.2 ppm and 159.8 ppm; aromatic, p-substituted carbons at 119.5 ppm, 119.8 ppm, 131.0 ppm and 131.5 ppm, respectively; aliphatic ether carbons were observable at 72.8 ppm with substantial shifts due to the terminal effects on the chemical shifts of these ether carbons. There were no observable nitrile groups. This spectrum confirmed the structure of the compound shown above.

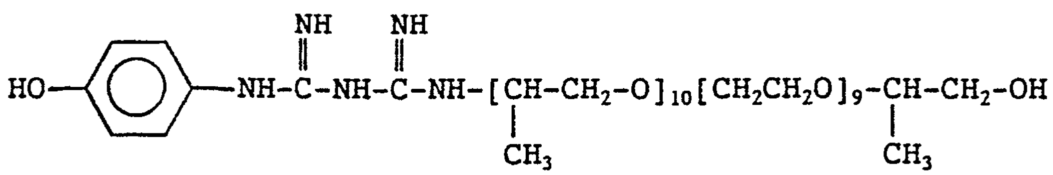

Example X

Polyether alkanol benzoate biguanide was synthesized by dispersing 5g p-cyanoguanidine benzoic acid (0.0208 mole) was dispersed in 18.93gm polyether alkanolamine and acidified to about pH 2.0. The reaction mixture was heated to 85°C under nitrogen as in the aforementioned example. Reaction progress was monitored by disappearance of the nitrile group at 2200 $cm^{-1}$. After ten minutes, the nitrile was no longer detectable and the product was isolated by dissolution in methanol, filtration and evaporation of solvent.

13 C-NMR spectroscopy confirmed the benzoic acid biguanidine was formed:

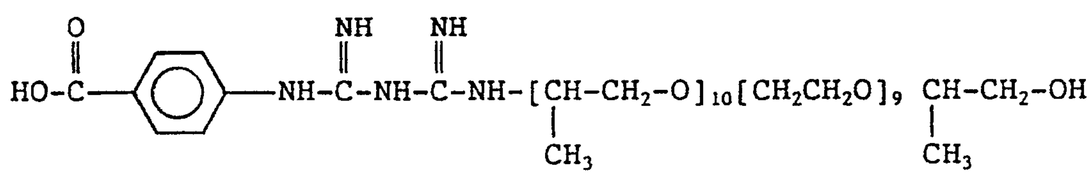

The spectrum showed the acid carboxyl at 171.9 ppm, one imine carbon at 158.19 ppm and another imine carbon at 156.4 ppm. The aromatic carbons at 140.16, 132.4, 134.3 and 128.5 respectively were clearly observable in the C-13 spectrum. A major C-13 resonance due to the ether carbons was observed at 72.33 ppm with some changes in minor amounts of the ether carbons due to the terminal effects in the polymer.

Example XI

Antimicrobial Evaluation

An antimicrobial evaluation was then completed on compounds having the following general formula

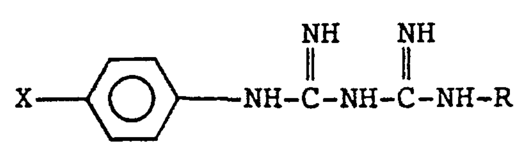

wherein R equals isododecyloxypropyl and X equals any number of moieties as indicated in Table I.

Antimicrobial evaluation of these compounds with minimum cidal concentrations of the various compounds against a number of microorganisms. In preparation of the evaluations, trypticase soy-yeast extract media was prepared at a 1:1 dilution and microorganisms were cultivated for 24 hours at 37° C. in this medium. Bacteria and yeast were thèn inoculated into the fresh media at an initial concentration of about 1 x $10^5$ cells/$ml^{-1}$. The cell suspensions were spiked with a range of biocides from 10 to 200 ppm and statically incubated at 37° for 24 hours. Cell growth was monitored by measuring the optical density of the media at 550 nm. After 24 hours exposure aliquots were inoculated onto the trypticase soy-yeast agar and incubated a further 24 hours. Colony counts were then performed to determine the remaining viable cell numbers.

The following microorganisms from the American Type Culture Collection were used: Listeria monocytogenes ATCC 19115, Salmonella typhi ATCC 6593, Staph. aureus ATCC 6538, Candida albicans ATCC 14055, Saccharouryces cerevisiae ATCC 9763, Esherichia coli ATCC 11229, and Pseudomonas aeruginose ATCC 15445.

TABLE OF BIGUANIDE BIOCIDAL ACTIVITY

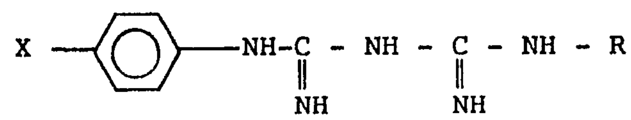

MINIMAL CIDAL CONCENTRATIONS (PPM)*

| MICROORGANISMS | $-NH_2$ (Ex.8) | $-CO_2H$ (Ex.4) | $CH_2$=CH-NH-CO- (Ex.7) | $CH_3$-OCO-$CH_2$-NH-CO (***) | OH- (Ex.2) | $CO_2H$ (**) | CONTROL (****) |
|---|---|---|---|---|---|---|---|
| L. monocyto. | >100 | 50-100 | 1-10 | 100-200 | 1-10 | >200 | 1-10 |
| S. typhi | 10-50 | 50-100 | 10-50 | 10-50 | ND | 50-100 | ND |
| S. aureus | 50-100 | 50-100 | 10-50 | 10-50 | 5-10 | >200 | 50-100 |
| C. albicans | 50-100 | 50-100 | 10-50 | 10-50 | 10-25 | >200 | 1-10 |
| S. cervisiae | 1-10 | 10-50 | 10-50 | 1-10 | 10-25 | >200 | 10-50 |
| E. coli | 50-100 | 100-200 | 10-50 | 10-50 | 10-25 | >200 | 10-30 |
| P. aeruginosa | 100 | >200 | 200 | >200 | 100-200 | >200 | 30-50 |

* ppm of active compound wherein x is indicated at the top of each column in the Table.

** R in this instance was $-(CH_2)_{11}CH_3$ monomer synthesized in accordance with Example 4 and not an ether amine.

*** Methyl ester synthesized from the intermediate of Example 5 in accordance with the process of Example VI.

**** The Control was chlorohexidine from Aldrich Chemical Company.

The foregoing specification, Examples and data provide a basis for understanding the invention. The invention can be made in a variety of embodiments without departing from the spirit and scope of the invention. Accordingly the invention resides in the claims hereinafter appended.

## Claims

1. An antimicrobial compound comprising:

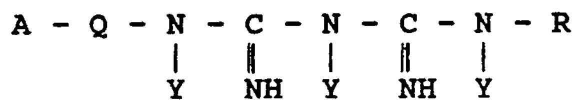

wherein Q = phenylene; Y = -H or -methyl;

R = $-(CF_2)_yCF_3$

wherein y = 1 - 20, $-(CH_2)_x-O-C_xH_{2x+1}$
wherein x = 1 - 25, $-(CH_2CH_2O)_n(CH(CH_3)CH_2O)_m$ $(CH_2CH(CH_3)O)_p(CH_2CH_2O)_qT$
wherein T = -H or a branched or unbranched $C_{1-20}$ alkyl,
n, m, p, q = 0 - 99 and n + m + p + q ≧ 1, -CHR'COOH wherein R' is a naturally occurring amino acid side chain, or $(CH_2)_xCOOH$; and A = -H, -OH, $-NH_2$, $-CH_2 = CH_2$, $-(CH_2CH_2O)_n(CH(CH_3)CH_2O)_m-(CH_2CH(CH_3)O)_p(CH_2CH_2O)_qT$, an amine protein linkage, an amine saccharide linkage, a polyhydroxyamine linkage or -COOX wherein X = -H, $-NR_1R_2$ with $R_1$, $R_2$ = H or alkyl, an amino alcohol moiety, an amino alkoxy silane moiety, $-CONH(CH_2)_xCOOH$, or $-NHCH_2CH-(OX')_2$ wherein X' = methyl or ethyl.

2. The antimicrobial compound of Claim 1, wherein Y = H; A = -OH or -COOH; and R' = H-, $CH_3$-,

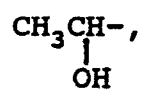

$CH_2(OH)$-, $H_2NCOCH_2$-, $H_2NCOCH_2CH_2$-, $CH_3CH(CH_3)$-, $CH_3CH(CH_3)CH_2$-, or

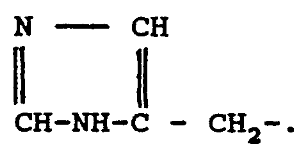

3. The antimicrobial compound of Claim 1 or 2 wherein R comprises $-(CH_2)_{10}COOH$.

4. The antimicrobial compound of Claim 1 wherein A comprises
a) an amino sugar selected from the group consisting of 2-dioxy-2-amino glucosamine, 2-dioxy-2-amino galactosamine or mixtures thereof; or
b) a polyhydroxy amine.

5. The antimicrobial compound of Claim 1, comprising:

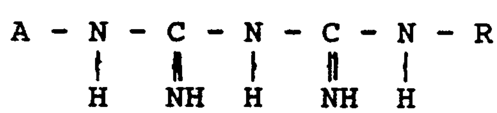

wherein A = -OH or -COOH; and R = $-NHCH_2CH-(OX')_2$

wherein X' = methyl or ethyl or $-(CH_2CH_2O)_n-(CH(CH_3)CH_2O)_m(CH_2CH(CH_3)O)_p(CH_2CH_2O)_q-H$; wherein n, m, p and q = 0 - 99 and $n + m + p + q \geqq 1$.

**6.** A protein having at least one substituent group comprising the compound according to Claim 1 wherein A comprises -

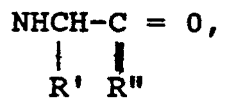

wherein R' is a naturally occurring amino acid side chain, and R" is the protein.

**7.** A polysaccharide, having at least one substituent group comprising the compound according to Claim 1 wherein A comprises

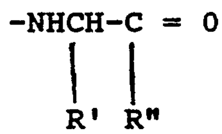

wherein R' is a naturally occurring amino acid side chain, and R" is the polysaccharide.

**8.** Use of the antimicrobial compound according to any one of Claims 1 to 5 in an adhesive comprising a binder composition and an antimicrobial effective amount of said compound, in a surface antimicrobial cleaner comprising an effective solvating amount of carrier and an antimicrobial effective amount of said compound, in a topical antimicrobial scrub comprising an effective cleansing amount of surfactant and an antimicrobial effective amount of said compound, in a coating comprising an effective filming amount of a film forming agent and an antimicrobial effective amount of said compound, in a personal care product comprising an effective moisturizing amount of conditioner and an antimicrobial effective amount of said compound, in a spray comprising an effective solvating amount of carrier and an antimicrobial effective amount of said compound or in a sealant comprising a sealing composition and an antimicrobial effective amount of said compound.

**9.** A film coated substrate having a coating wherein said coating comprises the compound of any one of Claims 1 to 5.

**10.** The film coated substrate according to claim 9, wherein said substrate is selected from the group consisting of woven or nonwoven fibers, porous or nonporous surfaces, or mammalian skin.